# EUROPÄISCHE PATENTSCHRIFT

(11) **EP 3 496 797 B1**
(45) Veröffentlichungstag und Bekanntmachung des Hinweises auf die Patenterteilung: **21.12.2022**
(21) Anmeldenummer: 17754607.4
(22) Anmeldetag: 02.08.2017
(51) Int. Cl.: A61M 25/09

(54) **FÜHRUNGSDRAHT ZUM EINSATZ IN SCHLAUCHFÖRMIGEN MEDIZINISCHEN SONDEN ZUR ERNÄHRUNGSTHERAPIE**
GUIDE WIRE FOR USE IN TUBULAR MEDICAL PROBES FOR NUTRITIONAL THERAPY
FIL-GUIDE À UTILISER DANS DES SONDES MÉDICALES TUBULAIRES POUR LA THÉRAPIE NUTRITIONNELLE

(30) Priorität: 12.08.2016 DE 102016009871
(43) Veröffentlichungstag der Anmeldung: 19.06.2019
(73) Patentinhaber: Häberle Laser- und Feinwerktechnik GmbH & Co. KG, 78713 Schramberg (DE)
(72) Erfinder: FRIETSCH, Thomas, 78655 Dunningen (DE) (DE)
(74) Vertreter: Lösch, Christoph Ludwig Klaus
(86) Internationale Anmeldenummer: PCT/EP2017/000937
(87) Internationale Veröffentlichungsnummer: WO 2018/028823

(56) Entgegenhaltungen:
- EP-A1- 1 040 842
- EP-A1- 1 195 174
- WO-A1-92/14508
- DE-A1- 19 823 414
- US-A- 4 390 017
- US-A- 4 676 249
- US-A1- 2002 049 392
- US-A1- 2005 049 523

## Beschreibung

Die Erfindung betrifft einen Führungsdraht zum Einsatz im Innern einer schlauchförmigen Ernährungs-Sonde, wie eine in der DE 10 2004 023 078 B3 (Absatz [0005]) erwähnt und in der US 4 390 017 A näher beschrieben ist.

Konventionelle schlauchförmige transnasale Magen-Sonden zur Nahrungs-, Flüssigkeits- und Medikamentenversorgung bestehen aus weichem Material, um Verletzungen der Speiseröhre beim Einführen des Sonden-Schlauches vorzubeugen. Der Vorschub eines solchen Schlauches von der Nase her weist nach der DE 2 97 18 991 U1, gegen ein Ausbiegen oder gar Abknicken des Schlauches im Zuge seines Vorschubes, eine Armierung in Form eines Drahtes auf, der achsparallel in die Wandung des Sonden-Schlauches eingeschoben oder eingeformt ist.

In der medizinischen Praxis ist es aber Standard, dass beim Einführen und zum Navigieren des Sonden-Schlauches als vorübergehende innere Armierung dem Vorschub des Sonden-Schlauches im Schlauch-Innenraum ein Führungsdraht im Pilgerschritt nachgeschoben wird. Das Vorschieben, Rückziehen und gegebenenfalls Drehen sowie Wieder-Vorschieben des Führungsdrahtes führt zum Plazieren des distalen Stirnendes der Sonde in ihrer Endposition im Magen oder im Darm über Verzweigungen hinweg, wie sie etwa im Nasenkanal oder im Magen anzutreffen sind. Anschließend kann der Führungsdraht wieder zurückgezogen werden, um den ganzen Schlauchquerschnitt insbesondere für Nahrungsmittel- oder Medikamentenzufuhren freizugeben.

Bei dem Führungsdraht handelt es sich gewöhnlich um einen massiven, säurebeständigen dünnen Edelstahl-Federdraht, um eine Drahtlitze oder um eine durchgehende hohlzylindrische Runddraht-Schraubenfeder, jeweils mit einer am distalen Stirnende gelegenen kugelförmigen Einführhilfe. Die vergleichsweise geringe Flexibilität des Führungsdrahtes beschwört allerdings die Gefahr herauf, im Zuge dessen Vorschiebens durch den Sonden-Schlauch diesen an Umlenkungen mit kleinem Radius oder gar Knickstellen zu durchstoßen. Dann kann der Versuch weiteren Vorschubes, aber auch ein Zurückziehen der Sonde, zu Verletzungen etwa der Speiseröhre eines Patienten führen. Bei einem als durchgehende Runddraht-Schraubenfeder ausgebildeten Führungsdraht tritt es abträglich auf, dass eine Zugkrafteinleitung zu einer Federdehnung führt, die bei schon weit vorgeschobener Sonde nicht mehr hinreichend abgebaut wird und so das endgültige Positionieren der Sonde erschwert oder gar verhindert.

Nach der eingangs zitierten, hier gattungsbildenden US 4 390 017 A ist der Sonden-Schlauch einer Ernährungssonde durch ein quecksilber-gefülltes flexibles Belastungs-Endstück verschlossen und proximal davor perforiert. Ein halbsteifer Führungsdraht ist von dem Sonden-Schlauch aufgenommen und kann nach dessem Positionieren aus ihm wieder zurückgezogen werden. Über einen distal letzten Abschnitt ist der Führungsdraht hochflexibel ausgelegt und distal mit einer kugelförmigen Einführhilfe bestückt. In diesem hochflexiblen Abschnitt distal vor dem halbsteifen Führungsdraht im Übrigen besteht allerdings die Gefahr des Abknickens im Sonden-Schlauch, ohne dass dieser biegeweiche Abschnitt ein korrigierend gegenwirkendes Richtmoment liefern kann.

In Erkenntnis derartiger Gegebenheiten liegt vorliegender Erfindung die technische Problemstellung zugrunde, einen Führungsdraht für eine Ernährungssonde zu schaffen, der einerseits die Gefahr eines Durchstoßens des Sonden-Schlauches minimiert und andererseits sich aus aktueller Verformung, auch noch bei kleinem Biegeradius oder langer Streckung, für möglichst störungsfreies weiteres Navigieren bis zu einem gewissen Maße selbsttätig wieder ausrichtet.

Das ist nicht dadurch erreichbar, dass, wie aus der WO 2011/038522 A1 zum Positionieren einer vaskulären Schlauchsonde bekannt, ein hohler Führungsdraht erstellt wird, der zu radialem Durchtritt von zu- oder abzuführendem Fluid proximal vor einer halbkugelförmigen Einführhilfe eine auf axiale Distanz gewickelte flexible Runddraht-Wendel distal vor dem hohlen Führungsdraht im Übrigen aufweist. Für die Navigation des Führungsdrahtes erstreckt sich, achsparallel durch dessen Hohlraum hindurch, zusätzlich ein Kerndraht durch die Drahtwendel bis in die Einführhilfe hinein. Diese, eine Halbkugel aus Kunststoff, ist vordem distalen Stirnende der Drahtwendel durch Press-Schweißen mit ihr verbunden.

Die Anforderungen werden auch nicht nach der DE 6 97 09 997 T2 erfüllt. Dort ist ein ebenfalls für einen vaskulären Katheter ausgelegter Führungsdraht längs des distalen Bereiches verjüngten Endabschnittes von einer auf große axiale Windungsabstände gewickelten Feindraht-Spule, insbesondere aus Platin, umgeben, um hier die Knickfestigkeit ohne signifikanten Verlust an Flexibilität zu erhöhen. Der eigentliche, hier verjüngte und von Gewebe ummantelte Führungsdraht erstreckt sich axial durch die Drahtspule hindurch bis zur Anlage gegen eine halbkugelförmige Einführhilfe, die stumpf vor den miteinander fluchtenden distalen Stirnenden von Drahtspule und Führungsdraht angeordnet ist.

Auch nicht um einen gattungsgemäßen Führungsdraht für eine Ernährungssonde handelt es sich bei dem Gefäßkatheder-System gemäß der EP 1 195 174 A1 = DE 6 0017 744 T2, bei dem ein mehrgängig aus Runddraht gewickelter Führungsdraht mittels eines darin gebogen verlaufenden Kernstabes durch das Gefäßsystem hindurch manövriert und positioniert wird; um erst anschließend z.B. einen intravaskulären Katheter-Schlauch längs des so fertig positionierten Führungsdrahtes über diesen hinweg mit dem Stirnende voraus direkt in die vom Kernstab vorgegebene Zielposition zu schieben. Ein vergleichbares vaskulär einzusetzendes System, mit aus Flachdraht gewickeltem Führungsdraht, wird schon in der EP 1 040 842 A1 = DE 6 99 17 213 T2 beschrieben. Bei beiden Varianten ist in Betracht gezogen, den gewickelten Führungsdraht einer Wärmebehandlung zu unterziehen, um ihn spannungsfrei zu machen. Mittels spanender Bearbeitung der Mantelfläche des jeweiligen Führungsdrahtes erhält dieser gestreckte Hohlkörper außen eine spitz-konische Form, die distal mittels einer pilzartigen Einführhilfe verschlossen ist. Andere Führungsdrähte für vaskulären Einsatz, wie sie in US 2005/0049523 A1, in WO92/14508, in US 2002/0049392 A1 oder in US 4,676,249 beschrieben werden, unterscheiden sich voneinander insbesondere durch konstruktive Auslegungen ihrer Spiralfedern, etwa zum Beeinflussen von deren richtungsabhängigen Biegesteifigkeiten. Der Führungsdraht gemäß DE 1 98 23 414 A1 weist, von einem sich verjüngenden Kern durchsetzte, ineinander gewickelte Spiralfeder-Gruppierungen auf, die distal stirnseitig von einem halbkugelförmigen Kopfteil verschlossen sind.

Nicht um einen Führungsdraht für vaskulären Einsatz, sondern um eine schlauchförmige Ernährungs-Sonde mit darin liegendem Führungsdraht handelt es sich bei der zuvor zitierten, nun gattungsbildenden US 4 390 017 A.

Die vorstehend genannte, ihr gegenüber zu lösende Aufgabe ist erfindungsgemäß durch die im unabhängigen Patentanspruch angegebenen Merkmale gelöst.

Danach ist zwischen der distalen Einführhilfe und dem Führungsdraht im Übrigen ein ihm gegenüber biegeweicherer, etwa 5cm langer Abschnitt eingefügt, der als unter Vorspannung auf Block gewickelte Flachdraht-Schraubenfeder ausgelegt ist.

Der distal vor dem Führungsdraht im Übrigen eingefügte Abschnitt mit gesteigerter Flexibilität ist also realisiert durch eine Schraubenfeder mit reduzierter Federkonstante im Vergleich zu derjenigen des sich proximal anschießenden herkömmlichen Führungsdrahtes im Übrigen, in Form einer Litze. Der distale, einerseits flexible und andererseits schubfeste Abschnitt am Ende des Führungsdrahtes fördert das Einführen der Sonde. Wenn der aktuelle Verlauf des Sensor-Schlauches relativ zum Führungsdraht zu einem von der Schlauch-Innenwandung auf die Einführhilfe ausgeübten radialen oder Quer-Druck führt, fördert ein Auslenken jenes Abschnittes vor dem Führungsdraht im Übrigen den Vorschub. Die verminderte Steifigkeit dieses Abschnittes reduziert die Anlagekraft der Einführhilfe gegen die Stützstelle im Schlauchinnern. Bei weiterem Vorschub kann die Einführhilfe in Richtung auf die Mitte des Schlauches ausweichen; die Gefahr, die Schlauch-Wandung hier von innen her zu durchstoßen, ist dadurch praktisch ausgeräumt.

Dabei wird berücksichtigt, dass der distale Endbereich des Führungsdrahtes jedoch nicht mehr ausweichen kann, wenn bei einem abgeknickten Schlauch dessen verbliebener innerer Querschnitt kleiner ist, als die Querabmessung der Einführhilfe. Dann führt weitere wiederholte Längsdruckausübung auf den Führungsdraht allenfalls zu einem geringfügigen elastischen Dehnen des Sonden-Schlauches; und jedenfalls der biegeweichere Abschnitt vor dem darauf folgenden Führungsdraht im Übrigen beult im Rahmen des im Schlauch-Innenraum hinter der Knickstelle verfügbaren Querschnittes elastisch bogenförmig radial aus, um schließlich auch den Schlauch entsprechend zu verformen. Infolge der damit sich bei der blockierten Einführhilfe einstellenden Kraftvektorzerlegung wird ein Großteil der auftretenden Kräfte über die Wandung des Schlauches abgeleitet; und nur noch ein so geringer Teil des auf den Führungsdraht zum Navigieren ausgeübten Längsdruckes wirkt sich auf die Einführhilfe aus, dass die Gefahr eines Perforierens der Knickstelle praktisch nicht mehr gegeben ist.

Die als biegeweicherer Abschnitt in den Führungsdraht distal eingefügte Schraubenfeder bietet, auf Block gewickelt, systembedingt generell den Vorteil, große axiale Kräfte übertragen zu können. Außerdem erfährt sie bei Querbeanspruchung (etwa infolge seitlichen Abstützens der Sonde gegen einen Bereich im menschlichen Körper), selbst nach einem Verlauf mit kleinem Biegeradius, keine bleibende Verformung, sondern sie kehrt nach Beendigung der Querkrafteinwirkung aus der belastungsbedingt abgewinkelt verlaufenden Form wieder in die ursprüngliche lineare Erstreckung zurück.

Dabei ist der hinter der Einführhilfe proximal sich anschließende, mechanisch geschwächte Abschnitt in Form einer biegeweicheren Schraubenfeder einlagig aus Flachdraht gewickelt, insbesondere aus gewalztem Flachdraht. Eine solche Schraubenfeder weist den zusätzlichen Vorzug auf, gegenüber der Runddrahtfeder aus vergleichbarem Drahtquerschnitt größere Zugkräfte bis zum Auftreten bleibender Verformung aufnehmen zu können. Das ist nützlich, wenn eine noch nicht endgültig positionierte Sonde sich im Körper verhakt hat und deshalb vom Führungsdraht unter Einsatz gesteigerter Zugkraft vorsichtig zurückgeholt werden muss. Die gesteigerte zulässige Zugspannung ergibt sich daraus, dass die Flachdraht-Schraubenfeder bei ihren axial aneinanderliegenden Windungen unter Vorspannung gewickelt ist. Eine auf axiale Distanz ihrer Windungen gewickelte Flachdraht-Schraubenfeder würde dagegen eine nur kleine Federkonstante aufweisen.

Zusätzliche Weiterbildungen und Abwandlungen der erfindungsgemäßen Lösung ergeben sich, auch unter Berücksichtigung von deren Vorteilen, aus nachstehender Beschreibung von in der Zeichnung annähernd maßstabsgerecht stark vergrößert skizzierten bevorzugten Realisierungsbeispielen zur Erfindung. In der Zeichnung zeigt, jeweils unter Beschränkung auf das Funktionswesentliche in abgebrochener Darstellung:
- Fig.1: im Sonden-Schlauch einen biegeweicheren da verschlankten Abschnitt von Massiv- oder Litzen-Führungsdraht als Übergang von einer Einführhilfe zum konventionellen Führungsdraht im Übrigen nach dem Stande der Technik und
- Fig.2: im Sonden-Schlauch eine Schraubenfeder als solchen biegeweicheren Abschnitt zur Erläuterung der Erfindung;
- Fig.3: eine exemplarische Flachdraht-Schraubenfeder als biegeweicheren Abschnitt zwischen distaler Einführhilfe und proximalem Anschluss an den Führungsdraht im Übrigen,
- Fig.4: als Einführhilfe einen kurzen, stirnseitig abgerundeten Runddraht in einer Flachdraht-Schraubenfeder;
- Fig.5: das Prinzip eines axial gegen eine perforationsgefährdete Umlenkung des Sonden-Schlauches abgestützten, distal geschwächten Führungsdrahtes und
- Fig.6: die Wirkung einer Flachdraht-Schraubenfeder in einer Situation etwa gemäß Fig.5.

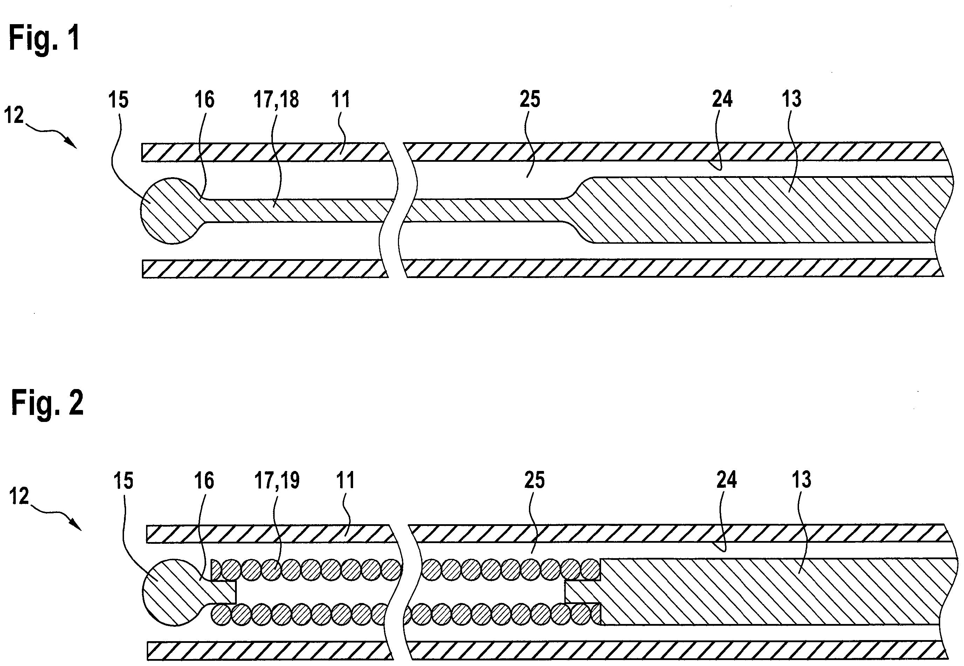

Der weiche Schlauch 11 gemäß Fig.1 etwa einer nasalen Magen-Ernährungs-Sonde 12 erfährt über seine Länge von typisch 50 bis 150 cm unter reichlich radialem Spiel gegenüber der Schlauch-Innenwandung 24 während des Einführens in den Patienten eine innere Armierung durch eine Litze oder durch einen massiven Federdraht aus säureresistentem Edelstahl als Führungsdraht 13 von einem Durchmesser im Bereich von typisch etwa 0,75 mm bis 1 mm. Das distale Stirnende 16 des Führungsdrahtes 13 ist mit einer stirnseitig abgerundeten Einführhilfe 15 versehen. Deren Durchmesser kann deutlich größer sein, als der des Führungsdrahtes 13, aber er ist kleiner als der Innendurchmesser des Schlauches 11; so dass - nach dem Navigieren der Sonde 12 - durch den Schlauch 11 Magensäure komplikationslos abgezogen werden kann, um die korrekte Sondenlage zu verifizieren. Für marktgängige Schläuche 11 mit 2 mm bis 3 mm Innendurchmesser beträgt der Durchmesser einer stirnseitig kugelförmigen Einführhilfe 15 etwa 1,3 mm. Auf die Einführhilfe 15 folgt proximal zunächst ein Abschnitt 17 reduzierter Biegesteifigkeit des Führungsdrahtes 13, ehe sich der steifere herkömmliche Führungsdraht 13 im Übrigen proximal anschließt.

Dieser typisch etwa 5 cm lange biegeweichere Abschnitt 17 zwischen der Einführhilfe 15 und dem Führungsdraht 13 im Übrigen ist mit einem, gegenüber der Stärke des Führungsdrahtes 13 im Übrigen, um etwa 20% bis 50% dünneren Einzel- oder Mehrfach-Draht 18 bestückt. Bei dem Draht 18 kann es sich z.B. um einen entspleißten zentralen Teil der Litzen des Führungsdrahtes 13 handeln, um einen massiven zwischen Einführhilfe 15 und Führungsdraht 13 im Übrigen eingeschweißten Edelstahl-Federdraht oder um eine Schraubenfeder 19 gemäß Fig.2.

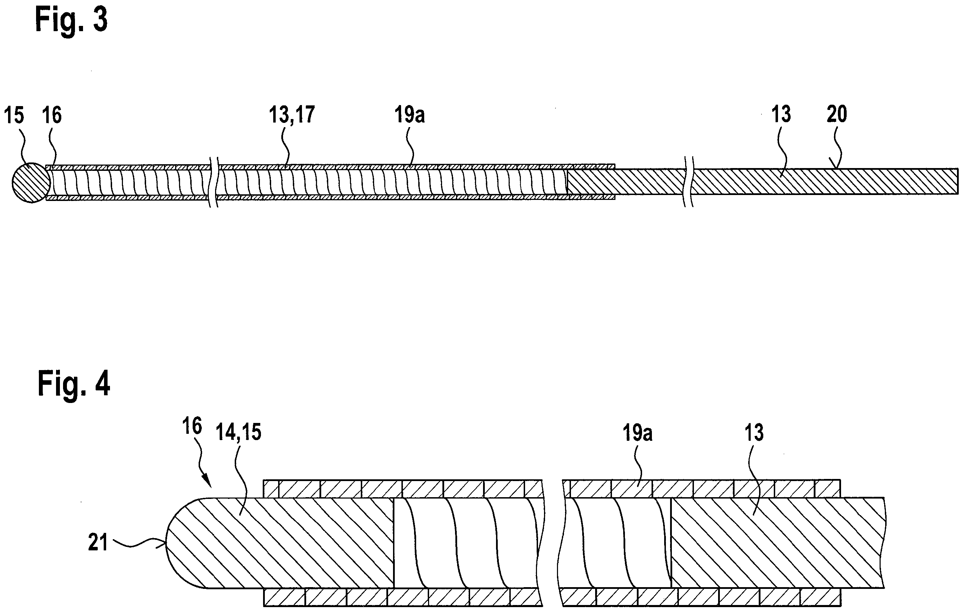

Erfindungsgemäß aber handelt es sich, um eine Flachdraht-Schraubenfeder 19a gemäß Fig.3, Fig.4 oder Fig.6.

Das freie Stirnende einer solchen auch so genannten Schlauchringfeder kann quer zur Längsachse angeschliffen sein, so dass die davor anzuschweißende ballige Einführhilfe 15 in Form einer vorgefertigten Stahlkugel selbstzentrierend in die distale Endwindung dieser Flachdraht-Schraubenfeder 19a eingreift.

Der Abschnitt 17 in Form einer derartigen Flachdraht-Schraubenfeder 19a ist beim Ausführungsbeispiel gemäß Fig.3 / Fig.4 auf der Außenmantelfläche 20 des Führungsdrahtes 13 im Übrigen mit seiner gegenüber dem Abschnitt 17 größerer Federsteifigkeit, kraft- oder stoffschlüssig festgelegt. Dafür greift der Führungsdraht 13 im Übrigen wie dargestellt mit seinem distalen Stirnende um einige Windungen in die Schraubenfeder 19a koaxial ein. Axial gegenüber wird der Außendurchmesser der Schraubenfeder19a im in Fig.3 beispielshalber dargestellten Falle einer distal davor befestigten kugelförmigen Einführhilfe 15 von dieser geringfügig radial übergriffen.

Gemäß Fig.3 / Fig.4 ist die Schraubenfeder 19a aus (gewalztem) Flachdraht, zwischen den aufeinanderfolgenden Windungen unter Vorspannung auf Block, gewickelt. Dann kann dieser Abschnitt 17 nicht so leicht unter Axialdruckbeanspruchung ausweichen. Andererseits erbringt das den Vorteil, dass unter Zugbeanspruchung für komplikationsloses Zurückziehen des Führungsdrahtes 13 aus dem positionierten Schlauch 11 heraus höhere Zugkräfte eingebracht werden können, ohne die Flachdraht-Schraubenfeder 19a dabei bleibend zu verformen.

Der Schweißvorgang zwischen den kleinen Massen zur Montage einer Einführhilfe 15 nach Fig.3 ist verfahrenstechnisch kritisch. Erfindungsgemäß ist daher vorgesehen, einen drahtförmigen flexiblen Abschnitt 17 gemäß Fig.1 distal zu verrunden. Einem weiteren Beispiel zufolge ist aber vorgesehen, in das distale Stirnende der Schraubenfeder 19a anstelle einer Kugel einen kurzen linearen Drahtabschnitt 14 (Fig.4) in der Größenordnung von 3 - 4 mm Länge eingreifen zu lassen und stoffschlüssig, insbesondere durch Schweißen koaxial darin festzulegen. Falls diese stiftförmige Einführhilfe 15 nicht schon derart abgerundet angeliefert wird, kann das distal vorragende Stirnende des Drahtabschnittes 14 problemlos nach der Montage durch Laserschmelzformen eine Verrundung 21 erfahren. Jedenfalls entfällt bei diesem Merkmal des Einsatzes eines kurzen linearen Drahtabschnittes 14 gemäß Fig.4 das Erfordernis eines Federanschliffes quer zur Längsachse, da keine Kugel mehr am distalen Ende der Schlauchringfeder zentriert werden muss.

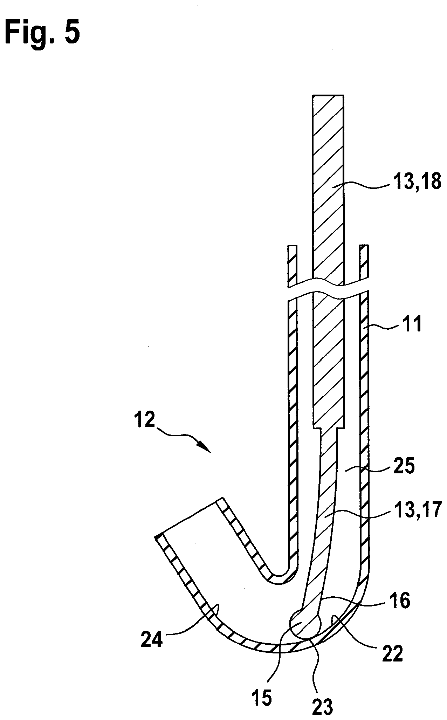

Sollte der Sonden-Schlauch 11 bei seinem Vorschub eine Umlenkung 22 etwa gemäß Fig.5 erfahren haben, dann besteht hier konventionell, an der Stützstelle 23, die Gefahr einer Perforierung durch die Einführhilfe 15 am Stirnende 16 eines relativ steifen und deshalb praktisch linear nachgeschobenen Führungsdrahtes 13 nach dem Stande der Technik.

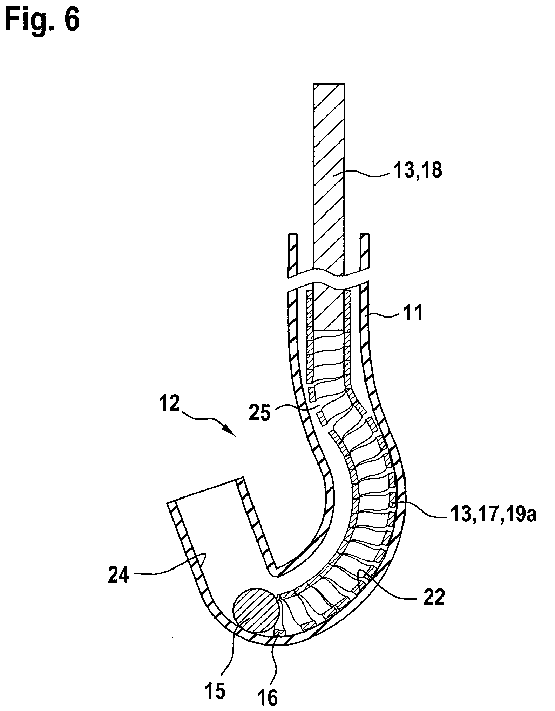

Diese Gefahr ist nun gebannt durch den erfindungsgemäß flexibleren Front-Abschnitt 17 des Führungsdrahtes 13; zumal, da gemäß Fig.6 auf die Einführhilfe 15 proximal der biegeweichere Abschnitt 17 des Führungsdrahtes 13 in Form einer Flachdraht-Schraubenfeder 19a folgt. Denn nun kann die Einführhilfe 15 bei externer axialer Druckausübung auf den Führungsdraht 13 dem Verlauf der Biegung der Sonde 12 längs der Schlauch-Innenwandung 24 in etwa folgen. Die Schraubenfeder 19a wird also elastisch verformt, was eine Rückstellkraft in die lineare Folge der Flachdraht-Windungen bewirkt; mit der Tendenz, den Verlauf der Umlenkung 22 zu begradigen. Auch der, dem Abschnitt 17 in die Sonden-Biegung 22 nachfolgende, dagegen biegesteifere Führungsdraht 13 im Übrigen übt auf diese Umlenkung 22 ein Richtmoment aus, das die Biegung in gewissem Maße begradigt, wenn er dem Abschnitt 17 nachgeschoben wird. Dabei liefert ein Abschnitt 17 in Form einer Schraubenfeder 19a ein größeres Richtmoment, als ein Federdraht oder gar ein Litzendraht, die stärkere bleibende Verformungen hinterlassen. Jedenfalls tritt infolge des Abschnittes 17 (Fig.6) praktisch keine perforationsgefährdete Stützstelle (23 in Fig.5) im Sonden-Schlauch 11 mehr auf.

Wenn aber einmal die Umlenkung 22 derart scharf verläuft, dass die Einführhilfe 15 diese Knickstelle, etwa infolge verringerten verbliebenen Durchmessers des Schlauch-Innenraumes 25, nicht mehr passieren kann, führt im Innern des Schlauches 11 die Abstützung der Einführhilfe 15 gegen die Knickstelle bei Längsdruck-Ausübung auf den proximalen Führungsdraht 13 gemäß Fig.6 zu einem Ausbeulen des Abschnittes 17 im Sonden-Schlauch 11 infolge Anlage gegen die Innenwandung 24 des Schlauches 11. Das hat zur Folge, dass Kraftkomponenten flächig radial auf die Wandung des Schlauches 11 übertragen und hierüber abgeleitet werden, so dass die verbleibende andere, lokal begrenzte Kraftkomponente gewöhnlich nicht mehr zu einem Durchstoßen des Schlauches 11 seitens der an der Knickstelle blockierten Einführhilfe 15 ausreicht.

Ein unter radialem Spiel zum Einsatz als Armierung in einem weichen Sonden-Schlauch 11 vorgesehener Führungsdraht 13 weist also proximal hinter einer Einführhilfe 15 zunächst einen, gegenüber dem Führungsdraht 13 im Übrigen biegeweicheren, Abschnitt 17 auf, ehe sich der konventionelle Führungsdraht 13 im Übrigen seinerseits proximal anschließt. Jener Abschnitt 17 kann derart ausgelegt sein, dass die Einführhilfe 15 und der herkömmliche Führungsdraht 13 im Übrigen dafür koaxial in einander gegenüberliegende Stirnenden einer mit Vorspannung gewickelten Schlauchringfeder, insbesondere einer unter Vorspannung auf Block gewickelten Flachdraht-Schraubenfeder 19a geringerer Biegesteifigkeit, eingreifen.

### Bezugszeichenliste

- 11: Schlauch (von 12)
- 12: Sonde
- 13: Führungsdraht (mit 17, 15; in 11)
- 14: Drahtabschnitt (mit 21; in 19/19a)
- 15: Einführhilfe (bei 16)
- 16: Stirnende (von 13, 17)
- 17: Abschnitt (verringerter Biegesteifigkeit; 13 proximal hinter 15)
- 18: Massivdraht (als 13, 17)
- 19: Schraubenfeder (als 17); 19a Flachdraht-Schraubenfeder (als 17)
- 20: Außenmantelfläche (von 13)
- 21: Verrundung (als 15)
- 22: Umlenkung (von 11)
- 23: Stützstelle (von 24 gegen 15 bei 22)
- 24: Innenwandung (von 11)
- 25: Innenraum (von 11)

## Patentansprüche

1. Zum Einführen in den Schlauch (11) einer Ernährungs-Sonde (12) ausgelegter, an seinem Stirnende (16) mit einer Einführhilfe (15) versehener Führungsdraht (13), wobei proximal hinter der Einführhilfe (15) ein Abschnitt (17) des Führungsdrahtes (13) vorgesehen ist, dessen Biegesteifigkeit geringer bemessen ist, als diejenige des sich ihm proximal anschließenden Führungsdrahtes (13) im Übrigen, **dadurch gekennzeichnet, dass** ein typisch etwa 5 cm langer biegeweicher Abschnitt (17) des Führungsdrahtes (13) zwischen der Einführhilfe (15) und dem Führungsdraht (13) im Übrigen durch eine aus Flachdraht unter Vorspannung auf Block gewickelte Schraubenfeder (19a) gebildet ist, an welcher distal die Einführhilfe (15) als Verrundung (21) ausgebildet ist und in welche proximal der Führungsdraht (13) im Übrigen, dieser eine Litze, koaxial eingreift und stoffschlüssig festgelegt ist.

## Claims

1. Guide wire (13) designed for inserting into the tube (11) of a nutritional probe (12) and provided at its end (16) with an insertion aid (15), wherein proximally behind the insertion aid (15) there is provided a portion (17) of the guide wire (13) of which the flexural stiffness is made to be less than that of the rest of the guide wire (13) proximally adjoining it, **characterized in that** a flexible portion (17), typically about 5 cm long, of the guide wire (13) between the insertion aid (15) and the rest of the guide wire (13) is formed by a helical spring (19a) wound from flat wire under prestress to form a block, on which distally the insertion aid (15) is formed as a rounding (21) and in which proximally the rest of the guide wire (13), the latter a strand, coaxially engages and is fixed in a material-bonding manner.

## Revendications

1. Fil métallique de guidage (13) conçu pour être inséré dans le tuyau (11) d'une sonde d'alimentation (12) et pourvu d'un auxiliaire d'insertion (15) à son extrémité frontale (16), une portion (17) du fil métallique de guidage (13) étant prévue du côté proximal en arrière de l'auxiliaire d'insertion (15), portion dont la résistance à la flexion est dimensionnée pour être inférieure à celle du fil métallique de guidage (13) qui lui est adjacent du côté proximal, **caractérisé par** ailleurs en ce qu'une portion flexible (17), typiquement d'environ 5 cm de long, du fil métallique de guidage (13) est par ailleurs formée, entre l'auxiliaire d'insertion (15) et le fil métallique de guidage (13), par un ressort hélicoïdal (19a) qui est enroulé sous précontrainte sur un bloc à partir d'un fil métallique plat et au niveau duquel l'auxiliaire d'insertion (15) est conçu comme un arrondi (21) du côté distal et dans lequel le fil métallique de guidage (13) s'engage coaxialement du côté proximal en formant par ailleurs un cordon et est fixé par une liaison de matière.
